(19) Europäisches Patentamt
European Patent Office
Office européen des brevets

(11) **EP 0 910 405 B1**

(12) **EUROPEAN PATENT SPECIFICATION**

(45) Date of publication and mention
of the grant of the patent:
**24.10.2001 Bulletin 2001/43**

(51) Int Cl.[7]: **A61K 39/21**, C07K 14/155,
G01N 33/569, G01N 33/543,
C12Q 1/68, C12Q 1/70

(21) Application number: **97916761.6**

(22) Date of filing: **14.03.1997**

(86) International application number:
**PCT/US97/04020**

(87) International publication number:
**WO 97/33615 (18.09.1997 Gazette 1997/40)**

(54) **VACCINE CONTAINING CAPRINE ARTHRITIS-ENCEPHALITIS VIRUS AND USE FOR IMMUNOPROTECTION AGAINST HIV-1 INFECTION**

CAPRIN ARTHRITIS-ENZEPHALITIS VIRUS ENTHALTENDE VAKZINE UND VERWENDUNG ZUR IMMUNSCHUTZ GEGEN HIV-1 INFEKTION

VACCIN CONTIENT LE VIRUS DE L'ARTHRITE/ENCEPHALITE CAPRINE ET L'UTILISATION POUR DONNER UNE IMMUNOPROTECTION CONTRE L'INFECTION DUE AU VIH-1

(84) Designated Contracting States:
**AT BE CH DE DK ES FI FR GB GR IE IT LI LU MC NL PT SE**

(30) Priority: **15.03.1996 US 616854
15.03.1996 US 616855**

(43) Date of publication of application:
**28.04.1999 Bulletin 1999/17**

(73) Proprietor: **UNIVERSITY OF SOUTHERN CALIFORNIA
Los Angeles, CA 90007 (US)**

(72) Inventors:
• **DOUVAS, Angeline
Pasadena, CA 91107 (US)**
• **EHRESMANN, Glenn
Altadena, CA 91101 (US)**

(74) Representative: **UEXKÜLL & STOLBERG
Patentanwälte
Beselerstrasse 4
22607 Hamburg (DE)**

(56) References cited:
**WO-A-96/00784          WO-A-96/30527**

• **KNOWLES D.P. ET AL.: 'Structure and Genetic Variability of the Envelope Glycoproteins of Two Antigenic Variants of Caprine Arthritis-Encephalitis Lentivirus' JOURNAL OF VIROLOGY vol. 65, no. 11, November 1991, pages 5744 - 5750, XP002039538**
• **CHEEVERS W.P. ET AL.: 'Caprine arthrtis-encephalitis lentivirus (CAEV) challenge of goats immunized with recombinant vaccinia virus expressing CAEV surface and transmembrane envelope glycoproteins' VETERINARY IMMUNOLOGY AND IMMUNOPATHOLOGY vol. 42, no. 3-4, 1994, pages 237 - 251, XP002039539**

SEQUENCE LISTING

    (1) GENERAL INFORMATION:

       (i) APPLICANT: Regents of the University of Southern California

       (ii) TITLE OF INVENTION: Caprine Arthritis-Encephalitis Virus Provides Immunoprotection Against HIV-1 Infection

       (iii) NUMBER OF SEQUENCES: 21

       (iv) CORRESPONDENCE ADDRESS:

(A) ADDRESSEE: Campbell & Flores LLP
(B) STREET: 4370 La Jolla Village Drive, Suite 700
(C) CITY: San Diego
(D) STATE: California
(E) COUNTRY: United States
(F) ZIP: 92122

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:

(A) APPLICATION NUMBER:
(B) FILING DATE: 14 March 1997
(C) CLASSIFICATION:

(viii) ATTORNEY/AGENT INFORMATION:

(A) NAME: Campbell, Cathryn A.
(B) REGISTRATION NUMBER: 31,815
(C) REFERENCE/DOCKET NUMBER: FP-US 2495

(ix) TELECOMMUNICATION INFORMATION:

(A) TELEPHONE: (619) 535-9001
(B) TELEFAX: (619) 535-8949

(2) INFORMATION FOR SEQ ID NO:1:

(i) SEQUENCE CHARACTERISTICS:

(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:1:

```
          Glu Arg Lys Arg Glu Gly Phe Thr Ala Gly
          1               5                   10
```

(2) INFORMATION FOR SEQ ID NO:2:

(i) SEQUENCE CHARACTERISTICS:

(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:2:

```
              Ser His His Gly Asn Asp Ser Arg Arg Arg
              1               5                   10
```

(2) INFORMATION FOR SEQ ID NO:3:

    (i) SEQUENCE CHARACTERISTICS:

        (A) LENGTH: 8 amino acids
        (B) TYPE: amino acid
        (D) TOPOLOGY: linear

    (ii) MOLECULE TYPE: peptide

    (xi) SEQUENCE DESCRIPTION: SEQ ID NO:3:

```
              Ser Arg Arg Arg Arg Arg Lys Ser
              1               5
```

(2) INFORMATION FOR SEQ ID NO:4:

    (i) SEQUENCE CHARACTERISTICS:

        (A) LENGTH: 9 amino acids
        (B) TYPE: amino acid
        (D) TOPOLOGY: linear

    (ii) MOLECULE TYPE: peptide

    (xi) SEQUENCE DESCRIPTION: SEQ ID NO:4:

```
              Arg Lys Glu Thr Gly Thr Leu Gly Gly
              1               5
```

(2) INFORMATION FOR SEQ ID NO:5:

    (i) SEQUENCE CHARACTERISTICS:

        (A) LENGTH: 13 amino acids
        (B) TYPE: amino acid
        (D) TOPOLOGY: linear

    (ii) MOLECULE TYPE: peptide

    (xi) SEQUENCE DESCRIPTION: SEQ ID NO:5:

```
          Arg Lys Lys Arg Glu Leu Ser His Lys Arg Lys Lys Arg
          1               5                   10
```

(2) INFORMATION FOR SEQ ID NO:6:

    (i) SEQUENCE CHARACTERISTICS:

(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO

```
Arg Met Gln Arg Lys Glu Arg His Lys
1               5
```

(2) INFORMATION FOR SEQ ID NO:7:

(i) SEQUENCE CHARACTERISTICS:

(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:7:

```
Val Arg Ser Ser Tyr Gly Ile Thr Ser Ala
1               5               10
```

(2) INFORMATION FOR SEQ ID NO:8:

(i) SEQUENCE CHARACTERISTICS:

(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:8:

```
Gly Arg Ile Lys Leu Gln Gly Ile Gly Gly
1               5               10
```

(2) INFORMATION FOR SEQ ID NO:9:

(i) SEQUENCE CHARACTERISTICS:

(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:9:

```
Gln Glu Ile Leu Glu Asp Trp Ile Gln Gln
1               5                   10
```

(2) INFORMATION FOR SEQ ID NO:10:

    (i) SEQUENCE CHARACTERISTICS:

       (A) LENGTH: 11 amino acids
       (B) TYPE: amino acid
       (D) TOPOLOGY: linear

    (ii) MOLECULE TYPE: peptide

    (xi) SEQUENCE DESCRIPTION: SEQ ID NO:10:

```
Lys Arg Ile Asn Asn Lys Tyr Asn Lys Asn Ser
1               5                   10
```

(2) INFORMATION FOR SEQ ID NO:11:

    (i) SEQUENCE CHARACTERISTICS:

       (A) LENGTH: 10 amino acids
       (B) TYPE: amino acid
       (D) TOPOLOGY: linear

    (ii) MOLECULE TYPE: peptide

    (xi) SEQUENCE DESCRIPTION: SEQ ID NO:11:

```
Asp Gly Leu Leu Glu Gln Glu Glu Lys Lys
1               5                   10
```

(2) INFORMATION FOR SEQ ID NO:12:

    (i) SEQUENCE CHARACTERISTICS:

       (A) LENGTH: 10 amino acids
       (B) TYPE: amino acid
       (D) TOPOLOGY: linear

    (ii) MOLECULE TYPE: peptide

    (xi) SEQUENCE DESCRIPTION: SEQ ID NO:12:

```
Ser Val Phe Pro Ile Val Val Gln Ala Ala
1               5                   10
```

(2) INFORMATION FOR SEQ ID NO:13:

    (i) SEQUENCE CHARACTERISTICS:

       (A) LENGTH: 8 amino acids

(B) TYPE: amino acid
(D) TOPOLOGY: linear

(ii) MOLECULE TYPE: peptide

(xi) SEQUENCE DESCRIPTION: SEQ ID NO:13:

```
                            Ala Ile Asp Ala Glu Pro Thr Val
                            1                   5
```

(2) INFORMATION FOR SEQ ID NO:14:

(i) SEQUENCE CHARACTERISTICS:

(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:14:

```
GCAGTTGGCA TATTATGCTA CTAC                                        24
```

(2) INFORMATION FOR SEQ ID NO:15:

(i) SEQUENCE CHARACTERISTICS:

(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:15:

```
CTTGTTGTAC TCTTTGTCCT AGTG                                        24
```

(2) INFORMATION FOR SEQ ID NO:16:

(i) SEQUENCE CHARACTERISTICS:

(A) LENGTH: 23 base pairs
(B) TYPE: nucleic acid
(C) STRANDEDNESS: single
(D) TOPOLOGY: linear

(xi) SEQUENCE DESCRIPTION: SEQ ID NO:16:

```
GAGCAGTAAG ACATATGGCG CAC                                         23
```

(2) INFORMATION FOR SEQ ID NO:17:

(i) SEQUENCE CHARACTERISTICS:

(A) LENGTH: 31 base pairs

(B) TYPE: nucleic acid
(C) STRANDEDNESS: single
(D) TOPOLOGY: linear

(xi) SEQUENCE DESCRIPTION: SEQ ID NO:17:

TGATGCATTT GTATAAGATA GTGTTAGCTT T                    31

(2) INFORMATION FOR SEQ ID NO:18:

(i) SEQUENCE CHARACTERISTICS:

(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:18:

GGATTTGAAC TACACCCGCA G                    21

(2) INFORMATION FOR SEQ ID NO:19:

(i) SEQUENCE CHARACTERISTICS:

(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:19:

CCTGTTGATA CTATGAACCC TAGAC                    25

(2) INFORMATION FOR SEQ ID NO:20:

(i) SEQUENCE CHARACTERISTICS:

(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO:20:

AAGAACCTAA GCATCCCGCA AC                    22

(2) INFORMATION FOR SEQ ID NO:21:

(i) SEQUENCE CHARACTERISTICS:

(A) LENGTH: 28 base pairs

(B) TYPE: nucleic acid
(C) STRANDEDNESS: single
(D) TOPOLOGY: linear

(xi) SEQUENCE DESCRIPTION: SEQ ID NO:21:


GTGATGTTCC CTAATTGCAA TTCTAGTC                                                          28


**Description**

BACKGROUND OF THE INVENTION

FIELD OF THE INVENTION

This invention relates generally to the fields of immunology and medicine and more specifically to methods for providing immunoprotection against HIV-1 infection.

BACKGROUND INFORMATION

The incidence of acquired immunodeficiency syndrome (AIDS) has reached an epidemic level, particularly in third world countries in Africa, Asia and the Caribbean. Despite the expenditure of billions of dollars for research to discover drugs to treat or cure the disease, however, only modest progress has been made in identifying drugs that can delay the progress of the disease.

The causative agent of AIDS is the human immunodeficiency virus (HIV-1). HIV-1 infects a particular cell type of the immune system, the T cell. Upon entering a T cell, the HIV-1 genomic DNA is incorporated into the T cell genome, where it directs synthesis of viral proteins. New copies of the HIV-1 virus then are released from the infected T cell and further infect additional T cells. Ultimately, the infected T cells die and the HIV-1 infected individual's immune system becomes depleted and cannot ward off subsequent infections. As a result, victims of AIDS typically die from infections that normally would cause, at worst, a mild illness in a healthy individual.

Initially, treatments for AIDS were limited to methods of treating the infections that resulted from the depleted immune response. Later, drugs such as AZT and ddI, which are known as nucleoside analogs, were identified that could interfere with replication of the HIV-1 DNA. These drugs, particularly in combination, can prolong the life of AIDS patients. More recently, a class of drugs called protease inhibitors have been reported to be even more effective in inhibiting HIV-1 replication. It is hoped that the protease inhibitors, perhaps in combination with the nucleoside analogs, will further prolong the life of AIDS patients and even result in cures.

While the use of drugs as described above provides a means to kill the virus in HIV-1 infected individuals, such drugs are useful only after a person has become infected; they have no enhancing effect on the immune system. Clearly, a more preferable approach to stifling the AIDS epidemic would be to prevent HIV-1 infection in the first place. Vaccines, which stimulate a person's immune response against the vaccinating agent, are the logical choice for preventing HIV-1 infection. For example, vaccines have been used to prevent or reduce the severity of various viral diseases, including polio, measles, smallpox and influenza. In addition, a vaccine can stimulate the immune system in individuals already infected with a virus.

Numerous approaches have been made to develop a vaccine that would increase a person's resistance to infection with the AIDS virus. However, while various types of HIV-1 vaccines have been designed and have been tested in clinical trials, each suffers from a serious limitation. For example, subunit vaccines, composed of portions of an HIV-1 protein, provide only transient or ineffective protection. One of the obstacles to vaccine development is that HIV-1 undergoes rapid mutation as it reproduces. As a result, an immune response that is stimulated against a particular HIV-1 protein or strain of HIV-1 is ineffective against a changed form of the virus. Furthermore, antibodies produced to portions of the HIV-1 surface, which would be most effective in a vaccine, in fact stimulate HIV-1 infection.

Attenuated vaccines, which consist of live but reproductively defective viruses, also have been proposed. However, there is justified concern for injecting such an HIV-1 virus into an individual, particularly an otherwise healthy person. Thus, a need exists for a vaccine that provides a broad based immune response against HIV-1 but does not carry the attendant risks and limitations associated with the use of HIV-1 as the vaccinating agent. The present invention satisfies this need and provides additional advantages.

Caprine arthritis-encephalitis virus (CAEV) is a retrovirus that is closely related to the human immunodeficiency virus (HIV-1), which causes AIDS in humans. CAEV is known to infect goats, where it causes various pathologic conditions, including arthritis and encephalitis. CAEV infection occurs world wide and can result in costly losses to the goat farming industry.

In many parts of the world, goat milk commonly is consumed and, particularly, in less developed countries, is an important source of nutrition. However, goat milk seldom is pasteurized in such countries prior to human consumption. Also, the consumption of raw goat milk is increasing in the United States due to its availability in health food markets. In accordance with the present invention, it now has been recognized that CAEV-like viral forms can infect humans (h-CAEV), particularly those individuals who are occupationally exposed to goats or consume raw goat milk. While it has not yet been determined whether CAEV can cause a pathologic condition in humans, the close relationship of CAEV and HIV-1 suggests that it would be best to minimize the occurrence of CAEV infection in humans.

Knowles et al. (J. Virol. (1991), vol. 65, p. 5744-5750) describe the structure of envelope genes from CAEV-63 and CAEV-Co and the results of antibody responses induced in goats by said genes.

Cheevers et al. (Vet. Immunol. Immunopathol. (1994), vol. 42, p. 237-251) report that immunisation with a recombinant vaccinia virus expressing glycoproteins encoded by the CAEV-63 envelope gene did not protect goats from inflammatory joint lesions after challenge with CAEV-63.

WO 96/30527, which is a prior art document in accordance with the stipulations of Article 54(3) EPC, discloses that modified peptide fragments contained in the principle immunodominant domain (PID) of the transmembrane protein (TM) encoded by the CEAV *env* gene are capable of modifying the immunologic properties of the PID domain and of the Env protein.

Although efforts have been made in the dairy industry to prevent spread of CAEV in goats, the infection of humans with human adapted forms was heretofore unknown. Therefore, a diagnostic method which applies to humans is necessary. Such a method of diagnosing CAEV infected humans would be useful for identifying CAEV infected blood supplies. In particular, it is important to have a method of detecting CAEV infection in the blood supply because encephalitis associated with CAEV infection may occur only in a very young child receiving infected blood through a transfusion. Unfortunately, no convenient method is available for identifying the presence of a CAEV infection in humans. Thus, a need exists for a simple and inexpensive method for diagnosing the presence of CAEV infection.

## SUMMARY OF THE INVENTION

The present invention provides a vaccine comprising a live caprine arthritis-encephalitis virus (CAEV), or a CAEV immunogen, preferably a human-CAEV, and a pharmaceutically acceptable carrier. The invention further relates to the use of a CAEV immunogen for the preparation of a medicament for use in the prophylaxis or treatment of HIV infection. In addition, the invention relates to the use of a lymphocyte contacted *in vitro* with a therapeutically effective amount of a CAEV immunogen for the preparation of a medicament for use in the prophylaxis or treatment of HIV infection. This medicament, which can be present in the form of a vaccine, is useful, for example, to stimulate an immunoprotective response against CAEV or a cross-protective immune response against human immunodeficiency virus-1 (HIV-1) in a human individual and to vaccinate a human, or other non-goat mammal against CAEV infection.

The invention also provides a method of producing a medicament for stimulating an immune response in an individual against HIV-1 by administering a CAEV immunogen to the individual. Such a method is useful, for example, to increase the resistance to HIV-1 infection of an individual not previously exposed to HIV-1 or to CAEV, respectively, or to reduce the severity of a pathology caused by HIV-1 in an HIV-1 infected individual. In addition, the invention provides a method of producing a medicament for stimulating an immune response against HIV-1 by contacting a lymphocyte with a CAEV immunogen *in vitro*.

## BRIEF DESCRIPTION OF THE DRAWINGS

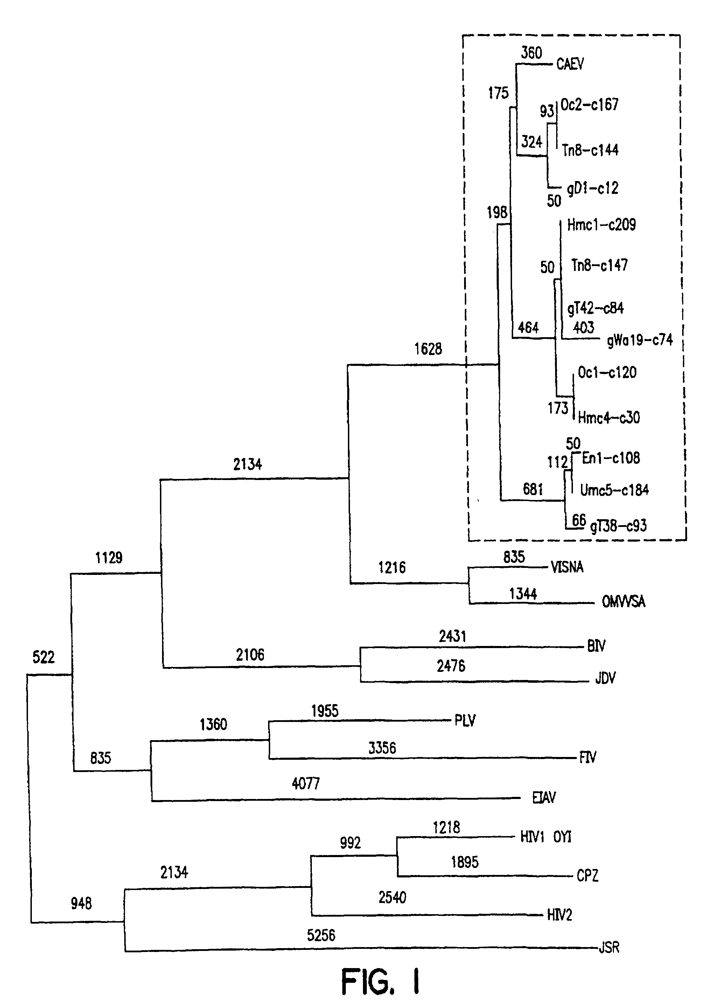

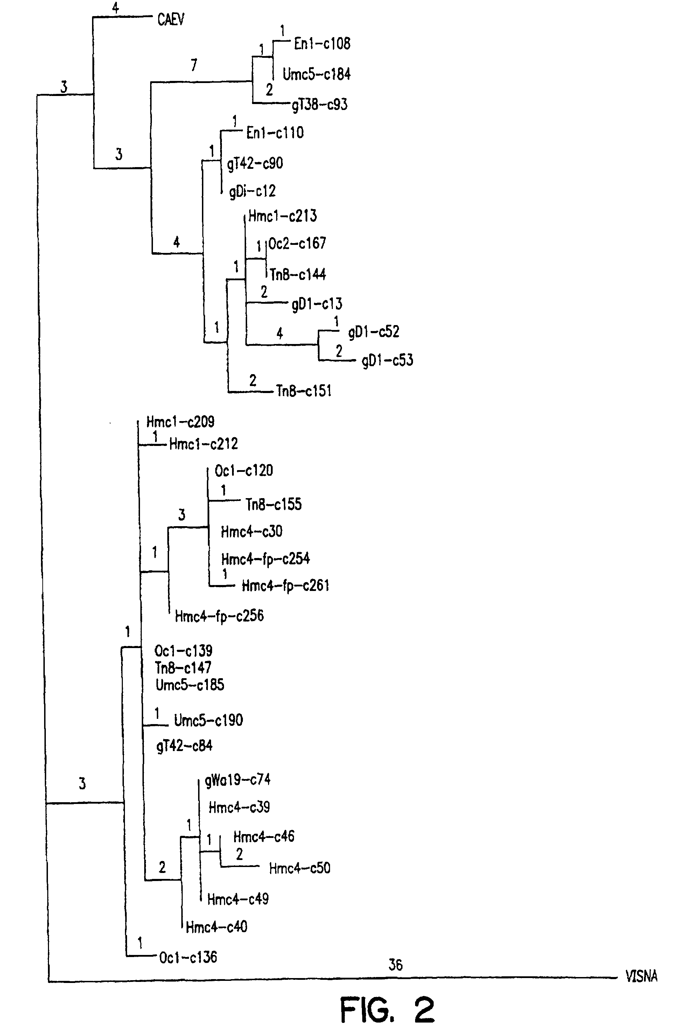

Figure 1 shows a phylogenetic tree analyses comparing CAEV gag sequences to other lentiviral gag sequences. PCR and RT-PCR, and cloning and sequencing of PCR products were performed as described in Example I.D. The various sequences analyzed, and their accession numbers, are as described in Example I.D. All CAEV-related sequences are enclosed in a box in Figure 1. All other sequences are lentiviral except for JSR, the Jaagsiekte sheep retrovirus, which serves as the designated ancestral sequence in Figure 1. The PAUP 3.1.1 parsimony algorithm was used to analyze 173 aligned gag sites, of which 157 wee varied in Figure 1 and 65 were varied in Figure 2. Randomization of the sequence input and MULPARS were two of the options employed. Inversely weighted parsimony was conducted in Figure 1, as described in Example I.D., yielding branch lengths that are not proportional to single nucleotide changes but which provide superior relative distances for the highly divergent sequences that are involved. One most-parsimonious tree was found in Figure 1, and its branching order was further supported by neighbor-joining analysis and bootstrapping, and by tree analyses utilizing pol gene fragments.

Figure 2 shows a phylogenetic tree analyses comparing CAEV gag sequences to one another as described in Example I.D. Ordinary parsimony is shown in Figure 2 (since these sequences are all closely related) and accordingly the branch lengths are given in units of minimal nucleotide changes. The maedi-visna sequence is the designated ancestral sequence. Two equally parsimonious trees were obtained in Figure 2 that differed in no substantive way.

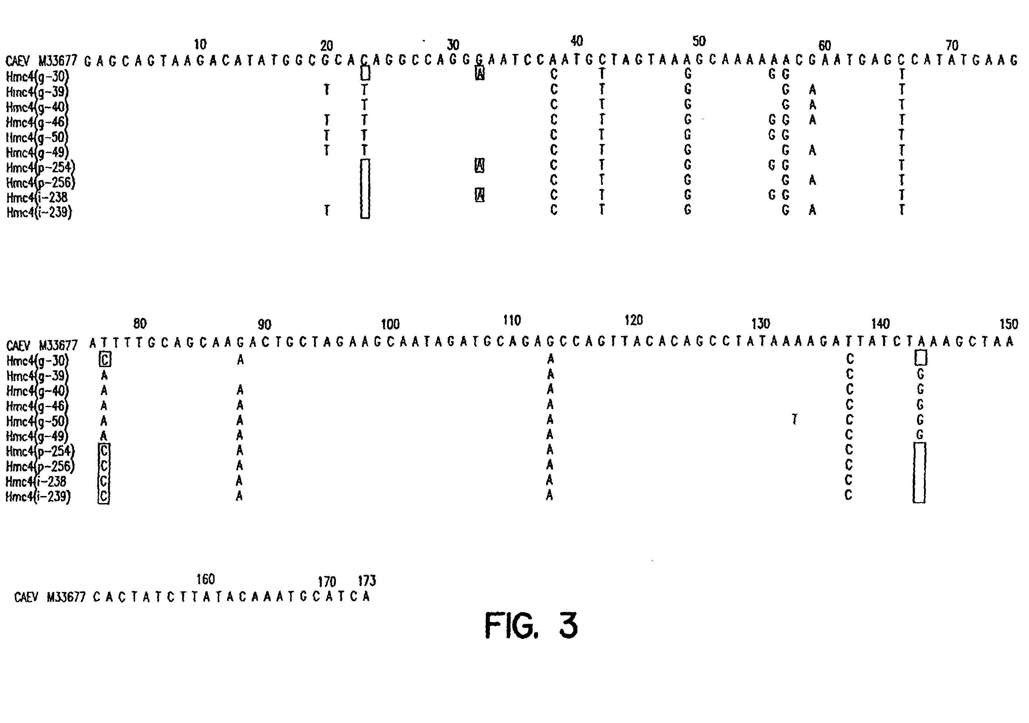

Figure 3 compares the nucleotide sequence of PCR apmplifed Hmc4-derived human-CAEV gag clones with the corresponding region of a goat infecting CAEV as described in Example II. The clones analyzed were Hmc4 genomic clones g-30, g-39, g-40, g-46 and g-50; Hmc4 plasma clones p-254 and p-256; clones from the infection experiment i-238 and i-239. The "i" clones were derived from the cytoplasm of infected cultures. The "p" clones were derived from the plasma of infected individuals.

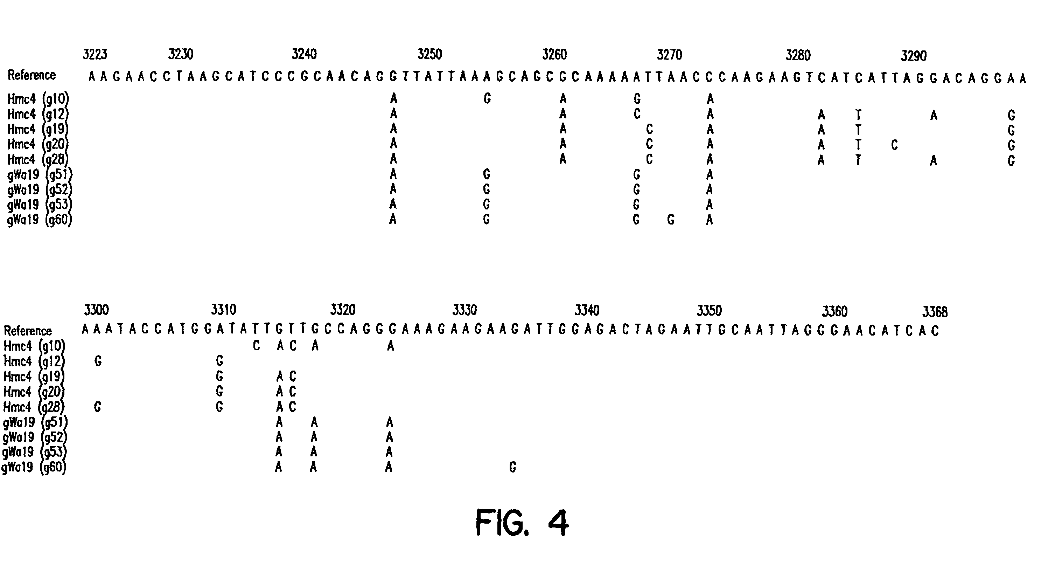

Figure 4 compares the nucleotide sequence of PCR apmplifed Hmc4-derived human-CAEV pol clones with the corresponding region of two goat infecting CAEVs as described in Example II.

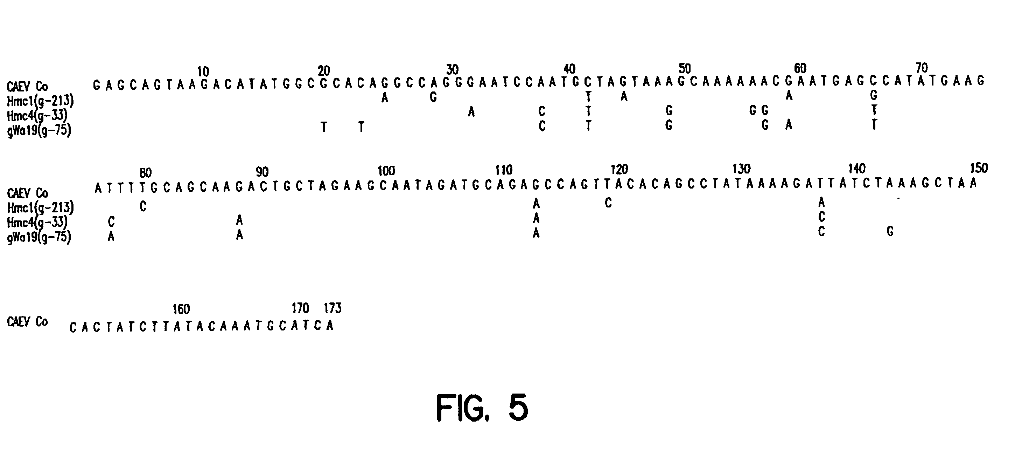

Figure 5 shows a direct sequence comparison of the goat-derived CAEV strains CAEV-CO and gWa19 (i.e.,

CAEV-79-63) and the human-derived h-CAEV strains Hmc1 and Hmc4.

## DETAILED DESCRIPTION OF THE INVENTION

The present invention provides a vaccine comprising a live caprine arthritis-encephalitis virus (CAEV), preferably a human adapted strain of CAEV, or a CAEV immunogen, and a pharmaceutically acceptable carrier. The present invention further relates to the use of a CAEV immunogen or the use of a lymphocyte contacted *in vitro* with a therapeutically effective amount of a CAEV immunogen, for the preparation of a medicament for use in the prophylaxis or treatment of HIV infection. As disclosed herein, the medicament, which can be a vaccine, is useful to stimulate an immunoprotective response against HIV-1 in a human individual. In addition, a vaccine of the invention is useful to vaccinate a human, or other non-goat mammal against CAEV infection.

As used herein, the term "CAEV immunogen" means a CAEV viral particle, which can be a live, attenuated or killed CAEV, or an immunogenic portion of a CAEV viral particle, which can be a portion of a CAEV viral particle or a peptide fragment of a CAEV protein such as a peptide fragment of the gp135 envelope glycoprotein (see Table 1; *env*). A CAEV immunogen is characterized in that it can stimulate an immune response, either *in vivo* or *ex vivo.* In particular, a CAEV immunogen can stimulate an immune response against CAEV when administered to a human and can stimulate an immune response that is cross-reactive against HIV-1. The cross-reactive immune response against HIV-1 that is generated by CAEV infection is reminiscent of the cross-reactive immune response against smallpox virus that is generated by cowpox infection.

TABLE 1

| CAEV IMMUNOGENS | |
|---|---|
| *env* | |
| | 26 ERKREGFTAG (SEQ ID NO: 1) |
| | 48 SHHGNDSRRR (SEQ ID NO: 2) |
| | 54 SRRRRRKS (SEQ ID NO: 3) |
| | 514 RKETGTLGG (SEQ ID NO: 4) |
| | 620 RKKRELSHKRKKR (SEQ ID NO: 5) |
| *pol* | |
| | 13 RMQRKERHK (SEQ ID NO: 6) |
| | 37 VRSSYGITSA (SEQ ID NO: 7) |
| | 83 GRIKLQGIGG (SEQ ID NO: 8) |
| | 307 QEILEDWIQQ (SEQ ID NO: 9) |
| | 1033 KRINNKYNKNS (SEQ ID NO: 10) |
| *gag* | |
| | 123 DGLLEQEEKK (SEQ ID NO: 11) |
| | 141 SVFPIVVQAA (SEQ ID NO: 12) |
| | 306 AIDAEPTV (SEQ ID NO: 13) |

- *env*, *pol* and *gag* refer to the envelope, polymerase and nucleocapsid core protein coding regions, respectively, of CAEV. Numbers preceding each sequence indicates the amino acid position in each CAEV protein that corresponds to the first amino acid shown for each peptide.

A peptide fragment of the 70K protein, which is present in the U1 ribonucleoprotein complex, that is homologous to an amino acid sequence present in HIV-1 can act as a surrogate immunogen, which stimulates an immune response that cross-reacts with HIV-1 (Douvas and Takehana, <u>AIDS Res. Hum. Retrovir.</u> 10:253-262 (1994)). As disclosed herein, various peptide fragments of CAEV proteins are homologous to 70K and HIV-1 sequences and, therefore, can be useful as CAEV immunogens for stimulating an immune response in an individual that cross-reacts with HIV-1.

Examples of peptide fragments of CAEV that are homologous to amino acid sequences present in 70K and HIV-1 are provided in the Table 1. Other exemplary CAEV peptides fragments contemplated herein with corresponding structural features in HIV-1(HIV-1-CAEV), include, for example: 220-FAILKC-200-FAILKC (hinge region); 99-DMVEQM-415-DMVEHM (α-helix 1); 129-LKCTDL-203-LKCTKW (conserved turn); 300-NNNTRT-480-NNNTIT (conserved turn), and the like. Still other such CAEV peptides useful as a CAEV immunogen can be identified by searching the CAEV DNA or amino acid sequence to identify CAEV peptides that are homologous to the immunologically homologous sequences of 70K and HIV-1 (see Douvas and Takehana, *supra,* 1994). Furthermore, methods for determining which CAEV peptides that are homologous to 70K and to HIV-1 peptides also are useful as CAEV immunogens are disclosed herein, including methods such as ELISA or western blot or viral neutralization assays (Example I), or otherwise known in the art (see, for example, Harlow and Lane, Antibodies: A laboratory manual (Cold Spring Harbor Laboratory Press 1988)).

As used herein, the term "vaccine" means a composition containing an immunogen which, upon administration to an individual, which generally is a non-goat mammal such as a human, and the like, stimulates an immune response in the individual. In particular, a vaccine of the invention contains a live CAEV or CAEV immunogen, which can be administered, for example, to a human. According to a preferred embodiment, a CAEV immunogen or a lymphocyte contacted *in vitro* with a therapeutically effective amount of a CAEV immunogen can be used for the preparation of a medicament for use in the prophylaxis or treatment of HIV infection, which can be administered, for example, to a human, wherein an immune response against CAEV that is cross-reactive against HIV-1 is stimulated. Thus, a CAEV immunogen is useful as a surrogate immunogen for stimulating an immune response against HIV-1. A particularly preferred use employs an attenuated human-CAEV, such as those set forth in Figures 3 and 4.

Although any CAEV infectious agent is contemplated for use herein, particularly preferred CAEVs for use herein are the novel human adapted strains of CAEV, referred to herein as "human-CAEV", such as the human strains obtained from patients Hmc1 and Hmc4 described in Example II, whose partial genomic sequence is set forth in Figures 3 and 4. It has been found that the human-CAEVs are distinct from the CAEVs able to infect goats. For example, within the genomic region corresponding to the 173 nucleotide fragment amplified by primers set forth in SEQ ID NOs: 16 and 17, it has been found that the this particular region of a human-CAEV virus has at least two mutations that are uniquely human in origin. These mutations correspond to a guanine at position 56 and a cytosine at position 77 within the 173 gag-region amplification product.

The human-CAEVs can be isolated by the methods described in Example III or employing methods well-known in the art.

It is recognized that a vaccine of the invention can be administered to an individual that is not infected with HIV-1, in which case the vaccine stimulates an immune response that can protect the individual from infection due to subsequent exposure to HIV-1, or can be administered to an HIV-1 infected individual, in which case the vaccine can stimulate an immune response so as to counter the immunopathology of HIV-1 (see, for example, Cease and Berzofsky, Ann. Rev. Immunol. 12:923-989 (1994)). Thus, a vaccine of the invention can be useful to increase an individual's resistance to HIV-1 infection or to reduce the severity of a pathology due to HIV-1 in an HIV-1 infected individual.

Prior to the present disclosure, it was not known that CAEV could infect humans. As disclosed herein, however, CAEV infection of humans is prevalent, particularly in populations where consumption of raw goat milk is common or where individuals in the population are otherwise exposed to goats (see Example I.C.). While it has not yet been determined whether CAEV infection causes a pathology such as arthritis or encephalitis in humans as it does in goats, if such a CAEV induced pathology exists, then it will be important to vaccinate persons involved in the goat farming industry against CAEV infection. A vaccine of the invention can be useful for this purpose.

Since it has now been determined that CAEV infects humans, it is recognized that there is a likelihood that CAEV also can infect other "non-goat" mammals, including, for example, non-human primates (e.g., monkeys, and the like), cows, horses, sheep, dogs, cats or other mammals. A vaccine of the invention can be useful for preventing or limiting the spread of CAEV infection in these non-goat mammals.

As disclosed herein, exposure of a human individual to CAEV also can stimulate an immune response to CAEV that is cross-reactive with HIV-1 (Example I.E.). CAEV is a retrovirus, subtype lentivirus, that is related to human immunodeficiency virus-1 (HIV-1). CAEV infects goats and causes arthritis and abnormalities of the immune system of infected animals (see, for example, Banks et al., Arthrit. Rheum. 30:1046-1053 (1987); Crawford et al., Science 207: 997-999 (1980)).

CAEV causes a persistent infection in goats and is associated with three disease syndromes, including arthritis, which occurs in 20-30% of infected animals; leukoencephalitis, which occurs in young animals; and sporadic neurologic disease, which occurs in adult goats. CAEV infection is found world wide and was identified as the cause of arthritis and encephalitis in goats in 1980. In 1985 and 1986, the relationship between the nucleotide and amino acid sequences of CAEV and HIV-1 (then called HTLV-III) was described. CAEV and HIV-1 are closely related phylogenetically and share a high degree of homology, including, for example, between their RNA dependent DNA polymerases (pol) and between gp120/41 in HIV-1 and gp135/38 in CAEV (see, for example, Gonda et al., Proc. Natl. Acad. Sci., USA 83:4007-4111 (1986); Gonda et al., Retroviridae 3:83-109 (1994); Garry et al., Retroviridae 4:491-603 (1995)).

CAEV is transmitted among goats through infected milk, particularly colostrum, and infection is spread by the agricultural practice of pooling colostrum to feed young animals. CAEV multiplies in cells of the monocyte/macrophage lineage and in fibroblast cell lines, but does not infect T cells. Macrophages expressing CAEV are distributed in the synovia, lungs, central nervous system, lymph nodes, spleen, gastro-intestinal tract and mammary glands of infected goats.

Prior to the present disclosure, CAEV was not known to infect humans. However, the detection of seroconversion by ELISA and western blot analyses using human blood samples (Example I.C.) and the detection of proviral DNA, corresponding to CAEV gag and pol genes, by polymerase chain reaction (PCR) analysis in genomic DNA obtained from an MCTD patient (Example I.D.) provides demonstrative evidence that CAEV infects humans. The incorporation of viral DNA sequences into host cell DNA is a hallmark of infection by the lentiviruses, including CAEV and HIV-1.

Based on the results disclosed herein, CAEV may infect as many as half the population of Mexico and Central America, particularly those who consume raw goat milk, which is common in these areas, or are otherwise exposed to goats (see Example I.C.). Although there is no evidence that CAEV causes a pathologic condition in humans, it is important, nevertheless, to determine the scope of CAEV infection in the human population in order to identify whether, for example, a pathology of unknown etiology correlates with CAEV infection. The ELISA assay provided herein allows for routine screening for CAEV infection in humans and provides the additional advantage of being useful for screening other non-goat mammals to identify the presence of CAEV infection.

PCR analysis also was performed on genomic DNA obtained from three groups of goats from different geographical regions and from a CAEV infected human (see Example I.D.). The PCR analysis of human and goat genomic DNA identified at least two potential strains of CAEV, which differ from the DNA sequence of a reference CAEV strain (reference CAEV DNA sequence is available as GenBank Accession No. M33677). Remarkably, the immune responses generated in two of the three groups of CAEV infected goats and in several CAEV infected human individuals were cross-reactive with HIV-1, indicating that either of these strains can be used as a source of a CAEV immunogen used in the invention.

CAEV infected goats have been used as an animal model for human rheumatoid arthritis, which is the most well known and prevalent syndrome in a cluster of arthritic/autoimmune disorders, the systemic rheumatic disorders. The systemic rheumatic disorders are about three times more common in women than men.

Mixed connective tissue disease (MCTD) is another systemic rheumatic disorder. MCTD is characterized by autoantibodies to the U1 snRNP splicing complex. MCTD is considered to be an overlap syndrome, in that it embraces clinical and serologic features common to three other systemic rheumatic disorders - systemic lupus erythematosus (SLE), scleroderma and polymyositis. The clinical profile of MCTD includes arthritis, lymphadenopathy, vasculitis, myositis, Sjogren's syndrome (lymphocytic infiltration of salivary and lacrimal glands) and immune dysregulation.

The clinical profile manifest in MCTD individuals also occurs in HIV-1 infected individuals. However, these clinical manifestations have a more severe course and are more refractory to therapy in HIV-1 infected persons. For example, Sjogren's syndrome in MCTD individuals is characterized by localized lymphocytic infiltration, whereas widespread glandular infiltration, referred to as diffuse infiltrative lymphocytosis syndrome, occurs in the corresponding HIV-1 associated syndrome.

Severe necrotizing vasculitis accompanied by neuropathy, inflammatory polymyopathy unrelated to drug therapy and other forms of vascular and neuromuscular disease associated with rheumatic disorders also occur in HIV-1 infected individuals. Necrotizing lymphadenopathy, which results in massive destruction of lymph node structure, occurs in MCTD, SLE and HIV-1 infection. Two classical rheumatic disorders, psoriatic arthritis and Reiter's syndrome, are the earliest manifestations of HIV-1 infection and are more refractory to chemotherapeutic agents when associated with HIV-1 infection.

The clinical similarities of HIV-1 infection and the systemic rheumatic disorders such as MCTD suggest that HIV-1 can be a precipitant of rheumatic pathology or that a phylogenetically related, but more clinically benign lentivirus is an etiologic factor in rheumatic disorders. Serologically, MCTD is characterized by the presence of anti-RNP (ribonucleoprotein) antibodies specific for the U1 snRNP particle, which is positioned at 5'-end of introns during nuclear RNA splicing. Anti-RNP antibodies also are present in some SLE and scleroderma patients. The U1 snRNP particle is composed of a core of U1 RNA and a cluster of RNA binding proteins, including the 70K, A and C proteins. Anti-RNP antibodies inhibit RNA splicing by binding U1 snRNP.

Although the humoral anti-RNP autoantibody response is pathognomonic in MCTD, the 70K protein, for example, contains both T cell and B cell epitopes and the humoral and cellular autoimmune manifestations are interdependent. T cell clones responsive to 70K epitopes have been isolated and include CD8+ T cells having cytotoxic T lymphocyte activity and CD4+ T cells having helper T cell activity. Some of the 70K T cell epitopes are homologous to T cell epitopes of HIV-1 (see Douvas and Takehana, *supra,* 1994). As disclosed herein, CAEV proteins, including CAEV gp135, also contain epitopes that are homologous to 70K and HIV-1 T cell epitopes (see Table 1, *supra*). Thus, a CAEV immunogen comprising such an epitope can induce a broad based cross-reactive immune response against HIV-1.

In view of the clinical similarities of HIV-1 infection and MCTD and the identification of CAEV infection in MCTD patients and in otherwise healthy individuals, it was important to determine whether individuals infected with CAEV develop an immune response that cross-reacts with HIV-1. The level of immunity to the HIV-1 protein, gp120, in blood obtained from CAEV infected individuals was examined by ELISA and by western blot analysis (see Example I.E.). In addition to exploring the relationship between CAEV infection and the development of an immune response to HIV-1, the relationship between CAEV infection and MCTD was investigated. Since some MCTD patients from Mexico or Central America have antibodies that react to HIV-1 gp120 and inhibit HIV-1 infectivity *in vitro,* it remained to be established whether CAEV infected persons that do not have MCTD also develop immunity to HIV-1.

As disclosed herein, CAEV infection in humans, whether suffering from MCTD or otherwise healthy, stimulated a humoral immune response that cross-reacted with the HIV-1 gp120 or p24 viral antigen (see Example I.E.). Significantly, none of the humans examined was infected by HIV-1 as determined using clinically certified assays. These results indicate that immunization of an individual with a CAEV immunogen can generate cross-reactive immunity to HIV-1, thereby increasing the resistance to HIV-1 infection in an uninfected individual or reducing the severity of a pathology due to HIV-1 in an HIV-1 infected individual. Furthermore, the presence of a systemic rheumatic disorder such as MCTD in a CAEV infected patient substantially increased the level of HIV-1 cross-reactivity (Example I.E.). Thus, administration of a CAEV immunogen, preferably in combination with an autoantigen associated with MCTD, such as a 70K immunogen, which generates a cross-reactive immune response against HIV-1 (Douvas and Takehana, *supra,* 1994), can be particularly effective in stimulating an immune response that cross-reacts with HIV-1 in a human individual.

A CAEV immunogen can be immunogenic by itself or can be attached to a carrier molecule such as bovine serum albumen or an inert carrier such that the CAEV immunogen-carrier complex can stimulate an immune response (see, for example, Harlow and Lane, *supra,* 1988). For example, where a vaccine contains the entire CAEV virus, the virus is immunogenic and can stimulate an immune response in a vaccinated individual. CAEV is readily available from the American Type Culture Collection (ATCC VR-905).

The CAEV immunogen used in the invention also can contain a peptide portion of a CAEV protein such as gp135 (see Table 1, *supra*). It is recognized that each of the peptides exemplified in the Table 1 or otherwise useful in the invention may not be immunogenic by itself. However, it is well known that such haptens can be attached to a carrier molecule so as to be rendered immunogenic (see, for example, Harlow and Lane, *supra,* 1988). An immune response can be stimulated *in vivo* or *ex vivo.* For example, immune cells, including T cells and B cells, can be obtained from a subject and placed in a tissue culture medium. The cells then can be contacted with a CAEV immunogen, which can stimulate the immune cells and induce a T helper cell and cytotoxic T cell immune response.

In addition to a CAEV immunogen, a pharmaceutically acceptable carrier is used. Pharmaceutically acceptable carriers are well known in the art and include aqueous solutions such as physiologically buffered saline or other solvents or vehicles such as glycols, glycerol, oils such as olive oil or injectable organic esters. If desired, a vaccine of the invention can contain a pharmaceutically acceptable carrier that is an adjuvant. Adjuvants such as alum or Freund's complete or incomplete adjuvant, or other proprietary adjuvants are known in the art and commercially available (Ribi Immunochem Research, Inc.; Hamilton MT). The addition of an adjuvant generally decreases the amount of a CAEV immunogen required to stimulate an immune response.

A pharmaceutically acceptable carrier also can contain a physiologically acceptable compound that acts, for example, to stabilize the CAEV immunogen or increase its absorption. Physiologically acceptable compounds include, for example, carbohydrates, such as glucose, sucrose or dextrans; antioxidants, such as ascorbic acid or glutathione; chelating agents; low molecular weight proteins; or other stabilizers or excipients. One skilled in the art would know that the choice of a pharmaceutically acceptable carrier, including a physiologically acceptable compound, depends, for example, on the route of administration of the vaccine and on the particular physico-chemical characteristics of the CAEV immunogen. Various routes for administering a vaccine to stimulate an immune response are known in the art and include, for example, intravenous, intradermal and subcutaneous injection, oral administration and transdermal administration.

If desired, a vaccine of the invention also can contain a 70K immunogen, or peptide fragment thereof, which is a surrogate immunogen that stimulates an immune response that cross-reacts against HIV-1 (Douvas and Takehana, *supra,* 1994). The addition of a 70K immunogen to the vaccine is advantageous because it is an autoimmune protein that represents an enrichment of sequences that can stimulate early shared clones, which are anti-70K latent T cells and B cells that can be rapidly activated to react with HIV-1 or can produce cross-reactive anti-gp120/41 antibodies that can neutralize HIV-1. The addition of a 70K immunogen also can be advantageous in that it can provide co-amplification or synergistic amplification of shared clones with gp120/41. In addition, immunogenic fragments of 70K that do not contain amino acid sequences that stimulate antibodies that mediate the deleterious effects associated with HIV-1 infection can be selected (Douvas and Takehana, *supra,* 1994).

A CAEV immunogen can be produced by various methods, including, for example, by recombinant DNA methods or by methods of chemical synthesis. For example, if a CAEV protein or a peptide fragment thereof is desired, the protein or peptide fragment can be produced by cloning the appropriate CAEV coding sequence into an expression vector such as a baculovirus vector and the protein or peptide can be expressed in and isolated from the appropriate host cell. The nucleic acid sequence of CAEV is available as GenBank Accession No. M33677 (see, also, Saltarelli et al., Virology 179:347-364 (1990); Knowles et al., J. Virol. 65:5744-5750 (1991)). In addition, it can be desirable to produce the recombinant protein or peptide in a mammalian cell, which can perform a desired post-translational modification. Appropriate expression vectors and host cells are well known in the art and commercially available, and the skilled artisan would know how to select an appropriate vector/host cell system based on a particular need.

Where the CAEV immunogen selected is a peptide immunogen such as, for example, the CAEV immunogens exemplified in the Table 1, the peptide can be synthesized using well known method of chemical synthesis, including, for example, automated solid phase methods. Chemical synthesis of a CAEV immunogen can be particularly desirable because the method allows for the introduction of amino acid analogs into the peptide. For example, it can be desirable to synthesize a CAEV immunogen containing one or a few (D)-amino acid substitutions for corresponding naturally occurring (L)-amino acids. The incorporation of a (D)-amino acid can confer desirable properties on the CAEV immunogen, including, for example, increasing the stability of the peptide, which can be particularly useful for preparing a vaccine that is to be distributed to regions of the world where refrigeration is not always available or dependable. In addition, a CAEV peptide can be synthesized and, if desired, can include an additional cysteine residue, which can facilitate specific attachment of the peptide to a matrix or to a carrier molecule such as keyhole limpet hemocyanin using appropriate oxidizing conditions.

The present invention also relates to stimulating an individual's immune response against CAEV or stimulating an immune response that is cross-reactive against HIV-1. Specifically, a CAEV immunogen is used for preparing a medicament for use in the prophylaxis or treatment of HIV infection. It can be advantageous to administer a 70K immunogen to the individual, either in combination with the CAEV or VMV immunogen or as a separate treatment.

Although reference is made herein to the administration of a CAEV immunogen to an individual, or to vaccination of an individual, it is recognized that an immune response against HIV-1 can be stimulated *in vivo* or *ex vivo.* Each of these methods is encompassed within the present invention. For example, it can be desirable to stimulate an immune response against HIV-1 *ex vivo* where the individual to be treated has AIDS. In this case, lymphocytes can be removed from the individual and immunized in culture. At the same time, the lymphocyte population can be expanded. The stimulated, expanded immune cells then can be reinfused into the individual, thereby providing a therapeutic advantage to the individual. Furthermore, even if the method for producing a medicament of the according to the invention is used in *ex vivo* immunization and reinfusion method which is not curative, such a treatment can be palliative and, therefore, can increase the quality of life of an individual suffering from AIDS.

As disclosed herein, vaccination of an individual with a CAEV immunogen stimulates an immune response that is cross-reactive against HIV-1. Vaccination against HIV-1 using a CAEV immunogen, either alone or in combination with a 70K immunogen, provides significant advantages over the use, for example, of a killed or attenuated form of HIV-1 or an HIV-1 protein antigen as a vaccine in that CAEV is not known to be a human pathogen and does not infect T cells. In addition, CAEV infection, particularly in individuals suffering from MCTD, provides a broad based immunologic resistance to HIV-1, including a B cell response and a T cell response against various strains of HIV-1. For example, in the population of Hispanic individuals examined in the study disclosed in Example I, greater than 60% of the individuals were infected with CAEV (see Example I.D.). In addition, 22% of the CAEV infected, but otherwise healthy, individuals demonstrated cross-reactivity to HIV-1 and 55% of the CAEV infected, MCTD patients demonstrated cross-reactivity to HIV-1 (Example I.E.). Controlled methods of immunization with a CAEV immunogen, alone or in combination with a 70K immunogen, can increase the frequency of individuals demonstrating cross-reactive immunity to HIV-1 (see Example II).

In order to stimulate an immune response against HIV-1, a therapeutically effective amount of a CAEV immunogen is administered to an individual. As used herein, the term "therapeutically effective amount" means an amount of an immunogen required to stimulate an immune response. The amount of an immunogen such as a CAEV immunogen that constitutes a therapeutically effective amount will vary, depending, for example, on whether stimulation of the immune response is *in vivo* or *ex vivo;* on whether the administration is a first administration or a booster administration; whether an adjuvant is administered with the immunogen; and, when administered *in vivo,* on the route of administration. In general, a therapeutically effective amount of a CAEV immunogen is about 50 μg to about 50 mg, preferably about 500 μg to about 5 mg. For example, where a CAEV virus is administered, a therapeutically effective amount of the particulate CAEV immunogen can be about 100 μg to about 2 mg, whereas, where a soluble CAEV immunogen is administered, a therapeutically effective amount can be about 1 mg to about 5 mg. Methods for determining a therapeutically effective amount of an immunogen are routine and well known in art (see Example II; see, also, Powell and Newman, Vaccine Design: The subunit and adjuvant approach (Plenum Publ. Corp.; 1994)).

Methods for vaccinating an individual so as to stimulate an immune response also are well known (Harlow and Lane, *supra*, 1988). For example, the immunogen can be administered intradermally, intramuscularly or intravenously. In addition, it can be advantageous to administer one or more booster immunizations. The need to administer a booster immunization and the timing of such booster immunizations can be determined experimentally by measuring, for example, the presence of anti-HIV-1 antibodies in a vaccinated individual's serum, using the methods disclosed herein.

The use of the invention can be useful for increasing the resistance to HIV-1 infection of an individual not previously exposed to HIV-1. Similarly, the vaccine of the invention can be useful for increasing the resistance to CAEV infection of a human or other susceptible non-goat mammal not previously exposed to CAEV. As used herein, the term "increasing the resistance," when used in reference to HIV-1 or CAEV infection, means that the likelihood of infection by the virus is reduced due to the stimulation of a immune response against the virus and consequent generation of memory immune cells, which can be rapidly mobilized against and sequester subsequent viral infection. In the case of CAEV, resistance is increased due to vaccination with a CAEV vaccine, whereas, in the case of HIV-1, resistance is increased to vaccination with a CAEV immunogen, which stimulates an immune response that cross-reacts with HIV-1.

In addition, the administration of a CAEV immunogen can be useful in an individual, including a human or a non-goat mammal, that currently is infected with a virus and is suffering a pathology due to the infection. In particular, a method of administering a CAEV immunogen to a human in order to stimulate an immune response in an HIV-1 infected individual that is cross-reactive against HIV-1 can be useful for decreasing the immunopathology of HIV-1. Specifically, the stimulation of such a response in an HIV-1 infected individual can prevent the further spread of HIV-1 infection in the individual, thereby reducing the depletion of T cells in the individual. Since many of the pathologies observed in an HIV-1 infected individual are due to the depleted immune system, a treatment that reduces depletion of the immune system can provide a therapeutic advantage.

Based on the studies disclosed herein, a significant population of persons, particularly in Mexico and Central America, may be infected with CAEV.

### EXAMPLE I

<u>CHARACTERIZATION OF HIV-1 CROSS-REACTIVITY IN CAEV INFECTED INDIVIDUALS</u>

This example describes the methods used to demonstrate that CAEV infected individuals generate an immune response that is cross-reactive with HIV-1 gp120.

<u>A. Human subjects:</u>

More than 50 human subjects were involved in the studies disclosed herein. The subjects were categorized based on their disease status (MCTD or normal) and their place of origin (Mexico/Central America or the United States) and were grouped as follows:

Group A. MCTD - Hispanic born females, clinically diagnosed with MCTD.

Group B. MCTD - U.S. born black or caucasian females, clinically diagnosed with MCTD.

Group C. MCTD - Guadalajara female residents, clinically diagnosed with MCTD.

Group D. Normal (non-MCTD) - Hispanic born females.

Group E. Normal - non-Hispanic males and females, including two workers in a veterinary service that treats infected goats.

Group F. Normal - 4 female and 4 male residents of a village located 30 km from Guadalajara, including the owner of a herd of 150 goats and 5 members of his family.

Group G. Scleroderma patients, all having anti-Scl-70/type 1 antibodies.

<u>B. Blood Samples and Reagents:</u>

Human and goat (see Section I.F., below) blood samples were collected and the serum component was used for immunologic testing. The antigens used in the ELISA and western blot assays to measure levels of serum antibodies included crude CAEV and affinity purified gp135 and recombinant HIV-1 gp120 and HIV-1 p24. Recombinant HIV-1 gp120 and p24 were purchased from Intracel (Shepreth UK).

Crude CAEV is prepared as described by Klevjer-Anderson et al., (<u>Virology</u> 110:113-119 (1981), which is incorporated herein by reference). Essentially, CAEV is grown in primary fetal goat synovial membrane (SM) cells grown in DMEM with a bicarbonate-HEPES buffer supplemented with 2 mM glutamine, 100 mg/ml streptomycin and 100 units/ml penicillin and containing 10% FBS. Medium of CAEV infected SM cultures is collected and clarified by centrifugation at 600 x g.

Aliquots of the crude CAEV preparation can be stored frozen at -70°C. The crude CAEV preparation can be used to isolate purified CAEV or can be used to prepare purified CAEV proteins. Purified CAEV proteins such as purified CAEV gp135 also can be prepared by cloning a nucleic acid molecule encoding gp135 (GenBank Accession NO. M33677; see, also, Saltarelli et al., *supra*, 1990; Knowles et al., *supra*, 1991) into an expression vector using routine methods of recombinant DNA technology and purifying the gp135 as described below.

CAEV gp135 is affinity purified from the crude CAEV preparation using an anti-gp135 antibody conjugated to a chromatography matrix. Essentially, the crude CAEV preparation is adjusted to 0.1% SDS to lyse the CAEV viral particles, then applied to an anti-gp135 antibody affinity chromatography column or recombinantly produced gp135 is applied to the affinity column.

Anti-gp135 antibody is isolated from CAEV infected goat serum or from serum obtained from goats immunized with a peptide fragment of CAEV gp135. If a CAEV infected goat is used as the source of serum, a goat having a high titer of anti-gp135 antibodies is used as the source of serum. Such serum can be identified by using western blot analysis to screen serum samples obtained from several different goats. Anti-gp135 antibodies are isolated from the serum by ammonium sulfate precipitation, followed by column chromatography to obtain the IgG fraction. The anti-gp135 antibodies are attached to a chromatography matrix such as cyanogen bromide activated Sepharose™. Methods for isolating IgG antibodies and attaching such antibodies to a matrix are well known and routine in the art (see, for example, Harlow and Lane, *supra,* 1988).

The crude CAEV preparation or recombinantly produced gp135 in PBS is loaded onto the anti-gp135 antibody affinity column and gp135 is allowed to bind to the antibodies overnight at 4°C. Following binding, the column is washed with PBS, then bound gp135 is eluted with 10 mM HCl, 0.15 M NaCl. Purity of the gp135 antigen is determined by SDS-PAGE and silver staining.

## C. CAEV ELISA and Western Blot Assays:

For ELISA assays, crude CAEV or affinity purified gp135 antigen was diluted 1:20 with phosphate buffered saline (PBS; final concentration gp135 approx. 1 µg/ml) and 50 µl was added to each well of a 96 well plate (Corning). The plate was covered and incubated overnight at 4°C, then antigen was removed and the wells were washed 3x for 1 min each with 90 µl washing solution (1 mg BSA/ml PBS). 100 µl blocking solution (10 mg BSA/ml PBS) was added to each well and plates were incubated 60 min at room temperature (RT), then the blocking solution was removed and the wells were washed 3x for 1 min each with washing solution.

Fifty µl of diluted serum sample (1:100 in washing solution) was added to each well and plates were incubated 60 min at RT. Blank (control) wells contained washing solution, alone. Following incubation, wells were washed as above, then 50 µl diluted second antibody was added and incubated as above. Where the serum sample to be analyzed was from humans, the second antibody was goat anti-human IgG conjugated to horse radish peroxidase (HRP; Zymed Laboratories, Inc.; South San Francisco CA; cat. # 62-8420) diluted 1:1000 in PBS. Where the serum sample to be analyzed was from goats, the second antibody was rabbit anti-goat IgG-HRP (Jackson ImmunoResearch Labs, Inc.; West Grove PA; cat. # 305035003, lot # 28671) diluted 1:3000 in PBS. Following incubation, wells were washed 2x with washing solution, then 2x with PBS.

Seventy-five µl color development solution (10 mg O-phenyldiamine (Sigma) in 25 ml ELISA buffer; 50 µl 30% $H_2O_2$, prepared fresh; ELISA buffer is 500 ml 0.1 M citric acid, 500 ml 0.2 M $Na_2HPO_4$, adjusted to pH 5.0) was added to each well and incubated 10 min at RT in the dark. Color development was terminated by adding 25 µl 6N $H_2SO_4$, then absorption was measured at $OD_{490}$.

For CAEV western blot analysis, 0.1-0.5 µg aliquots of gp135 in SDS sample buffer (Laemmli, Nature 227: 680-685 (1970), which is incorporated herein by reference), were separated by 10% SDS-PAGE, then transferred electrophoretically onto a nitrocellulose membrane. The membrane was incubated for 1 hr in 50 ml blocking buffer (1% BSA in WBS; WBS is 9 g NaCl, 1.21 g Tris-base, 0.25 ml NP-40 made up to 1 liter, pH 7.4), then washed 3x with washing buffer (0.1% BSA in WBS). The membrane was cut into strips, representing the lanes on the gel.

Serum samples were diluted 1:100 (20 µl serum/2 ml washing buffer; 40 µl/2 ml if plasma used), then added to an incubation tray containing a nitrocellulose strip and shaken for 60 min at RT. Following incubation, the strips were washed 3x with washing buffer, then 2 ml second antibody (as above, but diluted with washing buffer) was added and incubation continued for 60 min at RT. Strips were washed 3x with washing buffer and the enzyme reaction was initiated by adding 2 ml color development solution (10 mg 3,3-diaminobenzidine tetrahydrochloride (Sigma Chemical Co.; St. Louis MO; cat. # D 5905), 1 ml 0.05 M Tris, pH 7.5, 39 ml WBS, 80 µl $H_2O_2$, prepared fresh). Incubation was continued from 10 min at RT with gentle shaking, then color development was terminated by transferring the membrane to distilled water.

High reactivity to extracts of CAEV and to purified gp135 was observed in 62% of the serum samples obtained from Hispanic born MCTD patients (Table 2), including those from LAC/USC (group A) and those from Guadalajara, Mexico (group C). Serum samples from the disease control (group G) and from non-Hispanic healthy individuals, excluding the two veterinarians, were weakly reactive or non-reactive. The human serum samples that reacted to CAEV on western blots contained antibodies to the same polypeptides as those reacting with serum samples obtained from CAEV infected goats.

The reactivity to CAEV of serum samples from the normal individuals correlated with a history of drinking raw goat milk or other exposure to goats. The highest reactivities were present in the owner of the Guadalajara goat herd and in one of the veterinarians, who, in addition to treating CAEV infected goats, also reported regularly consuming raw goat milk. The second veterinarian, who also treated CAEV infected goats, also had positive reactivity.

In summary, 22 of 39 MCTD patients and 11 of 23 non-MCTD individuals had positive reactivity for CAEV infection, including 20 of 33 Hispanic MCTD patients (61%) and 9 of 14 Hispanic non-MCTD individuals (64%; see Table 2). These results indicate that over 60% of the Hispanic individuals examined in this study were infected with CAEV. In the CAEV reactive individuals, the reactivity of serum obtained from Hispanic MCTD patients was higher than that of the non-MCTD Hispanic individuals.

Table 2

| The relationship between CAEV gp135 and HIV-1 gp120 reactivity in MCTD patients and normals | |
| --- | --- |
| positive gp135 | negative gp135 |
| 1. MCTD | 3. MCTD |
| n=22; hispanic=20<br>total gp120(+) $\frac{12}{22}$ = 55%<br>hispanic gp.120(+) $\frac{12}{20}$ = 55%<br>2. NL<br>n=11; hispanic =9<br>total gp120(+) $\frac{4}{11}$ = 36%<br>hispanic gp120(+) $\frac{2}{9}$ = 22% | n=17; hispanic=13<br>total gp120(+) $\frac{0}{17}$ = 0%<br>hispanic gp120(+) $\frac{0}{13}$ = 0%<br>4. NL<br>n=9; hispanic=5<br>total gp120(+) $\frac{0}{9}$ = 0%<br>hispanic gp120(+) $\frac{0}{5}$ = 0% |
| Summary 1: CAEV(+) | |
| Total Hispanic<br>MCTD - hispanic<br>NL - hispanic<br>Total Nonhispanic<br>MCTD - nonhispanic<br>NL - nonhispanic | 29/47 = 62%<br>20/33 =61%<br>9/14 = 64%<br>2/15 = 13%<br>2/6 = 33%<br>2/6 = 33% |
| Summary 2: CAEV(+)/gp120(+) | |
| hispanic MCTD<br>hispanic NL<br>CAEV(-)/gp120(+)<br>hispanic MCTD and NL<br>non-hispanic MCTD and NL | 12/22 =55%<br>2/9 = 22%<br><br>0/20 = 0%<br>0/11 = 0% |

D. PCR analysis:

PCR analysis for identification of CAEV gag and pol genes was performed using genomic DNA isolated from peripheral blood mononuclear cells (PBMC). PBMC were obtained by Ficoll density gradient centrifugation of whole citrated or heparinized blood collected from selected human individuals and from WA, UC Davis and Gua goats (see below).

CAEV-specific genomic DNA sequences encoding regions of the gag protein were amplified in two stages. The first stage produced amplification products representing nucleotides 1057 to 1553 of CAEV. Primers were 5'-GCAGTTGGCATATTATGCTACTAC-3' (SEQ ID NO: 14; CAEV nucleotides 1057-1080) and 5'-CTTGTTGTACTCTTTGTCCTAGTG-3' (SEQ ID NO: 15; nucleotides 1530-1553). The second stage produced amplification products within the product of the first amplification product, from nucleotides 1329 to 1501. The primers for the second stage were 5'-GAGCAGTAAGACATATGGCGCAC-3' (SEQ ID NO: 16; nucleotides 1329-1351) and 5'-TGATGCATTTGTATAAGATAGTGTTAGCTTT-3' (SEQ ID NO: 17; nucleotides 1471-1501).

DNA encoding the CAEV pol also was examined by PCR analysis. Primers for the first stage of amplification were 5'-GGATTTGAACTACACCCGCAG-3' (SEQ ID NO: 18; CAEV nucleotides 2845-2865) and 5'-CCTGTTGATACTATGAACCCTAGAC-3' (SEQ ID NO: 19; nucleotides 3494-3518); primers for the second stage were 5'-AAGAACCTAAGCATCCCGCAAC-3' (SEQ ID NO: 20; nucleotides 3223-3244) and 5'-GTGATGTTCCCTAATTGCAATTCTAGTC-3' (SEQ ID NO: 21; nucleotides 3341-3368).

Amplifications were performed using an annealing temperature of 52°C and an elongation temperature of 72°C and were allowed to proceed for 38 cycles. Taq polymerase or pfu Taq polymerase (Promega Corp.; Madison WI) was used as recommended by manufacturer. One µl of the reaction mixture from the first stage of amplification was used for the second stage of amplification, which was performed under identical conditions. The products following the second stage of amplification were separated by agarose electrophoresis and collected by eluting the appropriate band. The amplified DNA samples then were cloned into a TA3 pCR™ vector (Invitrogen; La Jolla CA) and sequenced using a Sequenase™ Version 2.0 DNA sequencing kit (U.S. Biochemical Corp.; Cleveland OH) as recommended by the manufacturer. The cloned DNA sequences were compared to a reference CAEV DNA sequence (GenBank Accession No. M33677).

PCR analysis revealed that CAEV proviral DNA was present in genomic DNA of individuals that were CAEV positive as determined by ELISA or western blot analysis. Specifically, DNA encoding CAEV gag and pol sequences was amplified from genomic DNA of an MCTD patient (group A) and from the CAEV infected UC Davis goat. Comparison with the reference CAEV gag sequence demonstrated that the gag sequence in the MCTD patient diverged from the reference sequence by 8.2% and that the gag sequence in the UC Davis goat diverged by 8.8%. In addition, comparison with the reference CAEV pol sequence demonstrated that the CAEV pol sequence in the MCTD patient diverged by 7.6% and the CAEV pol sequence in the UC Davis goat diverged by 7.6%.

The relatively high degree of similarity between the CAEV sequences present in the human genomic DNA samples and the reference goat CAEV sequence provides definitive evidence of CAEV infection in humans. The average divergence of about 8% between the sequences indicates that the human CAEV infection can be due to a distinctive, human-adapted CAEV strain. Additional DNA sequencing can clarify whether a human-adapted CAEV strain is responsible for human CAEV infection.

To obtain further direct evidence for the presence of CAEV in human PBMC, viral *gag* and *pol* gene sequences were amplified by nested PCR from the DNA of 8 gp 135(+) subjects. CAEV *gag* was also amplified from cell-free plasma of the individual human subject Hmc4 by RT-PCR. Efforts to amplify CAEV gag and *pol* sequences from PBMC DNA of 11 gp 135(-) individuals were negative. All DNA samples used for CAEV *gag* and *pol* PCR were also subjected to PCR using HIV-1 *gag* and *pol* primers, and all were HIV-1 negative, except for the HIV(+) control group. CAEV *gag* sequences were also amplified from PBMC DNA of 4 gp 135(+) goats. Two CAEV gp135(+) goats (gD1 and gWA19) were from U.S. veterinary herds; gT38 and gT42 were Mexican gp135(+) goats. Twenty-two human PBMC *gag* clones, 3 HMC4 plasma *gag* clones and 11 goat PBMC *gag* clones were sequenced and compared to the published sequence of CAEV-CO *gag* (M. Saltarelli, et al., Virology, 179:347 (1990).

A phylogenetic analysis of 8 human *gag* sequences amplified from 7 individuals is shown in Fig. 1. Also included are 4 goat *gag* sequences. Clones and the corresponding Genbank accession numbers for CAEV, Visna and OMVVSA gag sequences analyzed in Figures 1 and 2 are: Hmc4-c30(U69922), -c39(U69923), -c40(U69924), -c46(U69925), -c49(U69932), -c50(U69926), -fp-c254(U69933), -fp-c256(U69934), -fp-c261(U69935); gD1c12(U69927), -c13(U69928), -c52(U69929), -c53(U69930); Tn8-c144 (U69931), -c147(U69909), -c151(U69910), -c155(U69911); gWa19-c74(U69918), -c75(U69919), -c77(U69920), -c80(U69921); gT42-c84(U69916), -c90(U69917); gT38-c93 (U69915); En1-c108(U69900), -c110(69901); Oc1-c120(U69905), -c136(U69906), -c139(U69907) ; Oc2-c167 (U69908); Umc5-c184(U69912), -c185(U69913), -c190(U69914); Hmcl-c209(U69902), -c212(U69903), -c213 (U69904); CAEV-CO (M33677); Visna (M18039); OMVVSA (M31646). All other sequences in Figure 1 are lentiviral sequences with exception of JSR, which is from the Jaagsiekte sheep retrovirus. In addition, CAEV pol clones and accession numbers corresponding to pol sequences discussed in the text are: Hmc4-c10(U69948),-c12(U69949), -c19(U69950), -c20(U69951), -c28(U69952); gD1-c1(U69944), -c2(U69945), -c3(U69946), -c30(U69947); gWa19-c51 (U69953), -c52(U69954), -c53(U69955), -c60(U69956) ; En1-c43(U69940), -c46(U69941), -c47(U69942), -c48 (U69943); Gmc15-c37(U69936), -c38(U69937), -c41(U69938), -c42(U69939).

The relative distances of human CAEV *gag* sequences from CAEV-CO *gag* and several other lentiviral *gag* sequences are made apparent using inversely weighted parsimony (D.M. Hillis et al., Science, 264:671 (1994). B.T. M. Korber et al., J. Virol., 68:6730 (1994)). Unweighted (ordinary) parsimony was performed using PAUP 3.1.1 (D.L. Swofford, Illinois Natural History Survey, Urbana). The twelve distinct substitution frequencies from the initial PAUP analysis were then ascertained using Macclade (W.P. Maddison and D.R. Maddison, "Macclade: Analysis of phylogeny and character evolution", Sinauer Associates, Inc., Sunderland, (1992)). An inverse weighting scheme was derived from this matrix of substitution frequencies which was then introduced into a subsequent parsimony analysis. The resulting branch length estimates are compound quantities generated from the matrix of inversely weighted substitutions. All viral *gag* sequences derived from experimental subjects and the goat *gag* sequences clustered with CAEV-CO *gag.*

Simple nucleotide distance measurements, i.e. Hamming distances, were determined using the SIMILARITY function of MASE (D.V. Faulkner and J. Jurka, Trends Biochem. Sci., 13:321, (1988)). Only single nucleotide differences were scored, gaps were not encountered in these data sets. By Hamming distance measurement, the range of sequence divergence of the 8 human *gag* clones from CAEV-CO *gag was* 5.3 - 9.4%, compared to 23.4 - 28% divergence from the published sequence of ovine maedi-visna *gag* (P. Sonigo et al., Cell, 42:369 (1985)).

To further assess inter- and intra-individual variation in human and goat gag sequences, an evaluation of 33 clones was performed by ordinary parsimony (Fig. 2). As in Fig. 1, all human sequences clustered with CAEV-CO. The mean sequence divergence of all 22 human *gag* clones from CAEV-CO was 8.4 ± 1.5%.

Viral sequences from 3 human subjects (Hmc4, Gmc15 and En1) and 2 goats (gD1 and gWA19) were also amplified from PBMC DNA using CAEV nested *pol* primers. The *pol* sequences were 4.8 - 8.2% divergent from the published CAEV-CO *pol* sequence (M. Saltarelli, et al., Virology, 179:347 (1990)) and 22.6 - 26.0% divergent from ovine maedi-visna *pol* (P. Sonigo et al., Cell, 42:369 (1985)). Parsimony analysis of these *pol* sequences yielded a topology consistent with that of *gag* (Fig. 1). Approximately 40% of *gag* and 17% of *pol* nucleotide changes resulted in amino acid changes. All of the *pol* changes and >80% of the *gag* changes were conservative (principally I↔V, K↔R and D↔E). Thus, evaluation of *gag* and *pol* sequences indicates that 8 CAEV gp 135(+) human subjects examined in this study harbor a ruminant lentivirus closely related to CAEV. Evidence for *in vivo* replication of this agent as described herein in Example II was obtained by RT-PCR amplification of CAEV *gag* sequences from cell-free plasma of subject Hmc4 (Fig. 2).

E. HIV-1 ELISA and Western Blot Assays:

Certified HIV-1 ELISA and western blot diagnostic kits were purchased from Organon Teknika (Cambridge UK) and the assays were performed as recommended by the supplier. Based on the clinical criteria using the certified HIV-1 ELISA and western blot assays, all of the human serum samples were negative for HIV-1, except for known HIV-1 positive sera and antibodies used as positive controls.

ELISA and western blot assays were performed using the individual recombinant HIV-1 antigens, gp120 and p24, and HRP-conjugated rabbit anti-goat and goat anti-human second antibodies (Zymed Laboratories Inc.) as previously described (Douvas and Takehana, *supra,* 1994; Crow et al., Cell. Immunol. 121:99-112 (1989), which is incorporated herein by reference). Subsets of sera that reacted with CAEV gp135 also reacted with HIV-1 gp120, including a total of 12 MCTD sera reacted with HIV-1 gp120 (see Table 2, *supra).*

As discussed in Section I.C., above, 22 of 39 MCTD patients and 11 of 23 non-MCTD individuals were positive for CAEV infection, including 20 of 33 Hispanic MCTD patients (61%) and 9 of 14 Hispanic non-MCTD individuals (64%; Table 2). The results of the HIV-1 assays further indicated that 12 of the 22 CAEV positive Hispanic MCTD patients (55%) and 2 of the 9 CAEV positive Hispanic non-MCTD individuals (22%) also showed positive reactivity to HIV-1 gp120 (see Table 2). In contrast, none of the CAEV negative Hispanic MCTD or non-MCTD individuals was positive for HIV-1 gp120.

These results indicate that CAEV infection of otherwise healthy individuals occurs without the concomitant development of clinical disease, including without developing symptoms of MCTD. However, exposure to CAEV results in the development of an immune response to CAEV that generalizes to HIV-1 in a subset of infected individuals. In addition, in CAEV positive MCTD patients, the reactivity to CAEV is about 1.6 times stronger than in CAEV positive non-MCTD individuals and the frequency of cross-reactivity to HIV-1 gp120 is more frequent. These results demonstrate that cross-reactivity to HIV-1 develops as a result of infection with CAEV and that the cross-reactive immune response is increased in an individual with an autoimmune disease.

F. CAEV Infected Goats:

Three groups of goats were studied as follows:

Group I. WA - uninfected goats and experimentally infected (CAEV) goats maintained at Washington State University, Pullman WA.

Group II. UC Davis - a naturally infected (CAEV) goat maintained at the University of California, Davis CA.

Group III. Gua - 20 naturally infected (CAEV) goats belonging to a goat herder in Guadalajara Mexico (see Group F, above).

Serum samples collected from the various CAEV infected or uninfected goats were examined. The Gua goats (Group III) lacked reactivity to CAEV gp135 but about 50% were reactive against CAEV gp38 and gag. In addition, the Gua goats, but not the WA goats showed strong reactivity to HIV-1 gp120 and p24. The UC Davis goat was reactive against both gp135, gp120 and p24.

These results demonstrate that differences exist between the immunologic reactivities of the WA and Gua goats to CAEV and to HIV-1 antigens. These differences may derive from different viral strains or may be due to the different routes of infection. For example, the WA goats were infected intravenously, whereas the Gua goats and the UC Davis goat were naturally infected via ingestion of infected milk.

G. HIV-1 Viral Neutralization Assay:

Virus neutralization assays are performed in 48 well tissue culture plates using PHA-activated normal donor PBMC maintained in growth medium at a concentration of $1 \times 10^6$ cells/ml as targets for infection. Various dilutions of HIVIG™, heat-inactivated (56°C, 30 min) MCTD sera or purified IgG are incubated with 10 or 50 TCID$_{50}$ (tissue culture infecting dose-50) of virus for 30 min at 37°C.

Negative controls for the MCTD sera and for HIVIG™ include heat inactivated serum from individuals not infected by HIV-1 and not affected by a systemic rheumatic disorder, and IVIG™, respectively. HIVIG™, which is a neutralizing IgG preparation from pooled serum from 9 seropositive donors in early stages of HIV-1 infection, and IVIG™, which is IgG from normal donors, are obtained from North American Biologicals Inc. (Miami FL). TCID$_{50}$ is determined using routine methods of viral titering.

Following incubation of the serum or IgG and the virus, serum/IgG-virus mixtures are added to the target cells in triplicate. After an overnight incubation at 37°C, the plates are centrifuged at 1000 rpm for 5 min and the medium is completely replaced. This washing step is repeated 3x to ensure complete removal of the serum/IgG-virus inoculum. On day 4 of culture, the medium is changed and on day 7 the supernatant is harvested for quantitation of p24 core antigen. Percent inhibition is determined by comparing the mean p24 core antigen concentration in each serum/IgG treated tissue culture well with that of the specific negative control.

In parallel, a control is prepared to measure any non-specific reduction in the amount of p24 core antigen measured due to a serum or HIVIG™ not being completely removed by the washing steps. At the time that the serum/IgG-virus mixtures are added to the target cells, each serum, IVIG™ or HIVIG™ also is added to target cells without preincubation with virus. These wells are treated in parallel with the experimental cultures. After 7 days of culture, the supernatant from each well is mixed 1:1 with that of the negative control and the p24 antigen concentration is quantitated. A measurement that is less than 50% the value of the negative control is considered non-specific inhibition of p24 antigen, which can be due to the presence of residual p24 antibodies.

The results of such an experiment are shown in Table 3. Table 3 indicates that anti-RNP sera from MCTD patients was able to neutralize infection by HIV-1 strains MN, JR-FL, and CO. Sample # 1 in Table 3 corresponds to the CAEV(+) (i.e., CAEV-infected) patient Hmc1. Sample # 6 in Table 3 corresponds to the CAEV(+) patient Hmc4. The patient corresponding to sample # 4 in Table 3 was also determined to be CAEV(+). The patients corresponding to samples 2, 3, 5 and 7 could not be assessed for CAEV-infectivity. Thus, since samples 1, 4 and 6 correspond to anti-CAEV sera from humans, it is apparent that human anti-CAEV sera neutralizes HIV-1 infection.

TABLE 3

| NEUTRALIZATION OF HIV-1 STRAINS BY ANTI-RNP SERA | | | |
|---|---|---|---|
| | | Strains | |
| Antibody | MN | JR-FL | CO |
| a. anti-RNP | | % neutralized | |
| 1 | 99 | 77 | 86 |
| 2 | 71 | 71 | - |
| 3 | 52 | 80 | - |
| 4 | 72 | 55 | 37 |
| 5 | 9 | 79 | - |
| 6 | 28 | 60 | 31 |
| 7 | 18 | - | - |
| 8 | 0 | - | - |
| 9 | 0 | - | - |

TABLE 3 (continued)

| NEUTRALIZATION OF HIV-1 STRAINS BY ANTI-RNP SERA | | | |
|---|---|---|---|
| | | Strains | |
| b. HIV-1(+) | | | |
| Pt. M | 99 | 30 | - |
| HIVIGtm | 93 | 94 | 54 |
| c. Normal | | | |
| NL-1 | 11 | - | - |
| NL-2 | 8 | - | - |
| NL-3 | 1 | - | - |
| IVIGtm | 19 | 20 | - |

**EXAMPLE II**

In vitro infection of human PBMC with PCR(+) plasma from a human subject

This example demonstrates the ability of h-CAEV to infect human PBMC cells. A comparison of rescued CAEV gag sequences will be presented.

Cultures of 0.8 million human PBMC from a donor who was negative for CAEV infection, as determined by PCR (i.e., PCR-), were inoculated with 100 µl of plasma from patient Hmc4, who was shown to be positive for CAEV infection (PCR+), in a total volume of 1 ml. After 4 days, the genomic DNA, cellular RNA and culture supernatant RNA were extracted and analyzed by PCR and RT-PCR using CAEV gag primers, as was 100 µl of the original plasma, diluted to 1 ml. Agarose gels of the PCR reactions show CAEV gag signals in the genomic DNA, RNA and culture supernatants, while levels of CAEV in the inoculating plasma of the patient Hmc4 were below the level of detection. This indicates that CAEV productively infected human PBMC cells. A similar infection of human PBMC with PCR(+) plasma from a human subject designated Hmc1 was performed with similar results.

CAEV virus was rescued from infected cell cultures in the above-described experiment. The sequences rescued from the Hmc4 experiment are compared to those of the genomic DNA and plasma RNA from the same subject. CAEV gag sequences cloned from the infected PBMC match those derived from the concentrated plasma of patient Hmc4 as shown in Figure 3. Figure 3 compares the following Hmc4-related CAEV gag clones, derived by PCR or RT-PCR: Hmc4 genomic clones g-30, g-39, g-40, g-46 and g-50; Hmc4 plasma clones p254 and p-256; clones from the infection experiment i-238 and i-239. The "i" clones were derived from the cytoplasm of infected cultures. The boxes indicate that there are certain similarities between all of the "p" and "i" clones, and genomic clone g-30 from Hmc4, suggesting that there is a subset of particularly viable forms in this patient.

From the data presented in Figure 3, it is readily apparent that CAEV subtype that infects humans is distinct from the reference goat-infecting CAEV M33677 (also referred to herein as "CAEV-CO"). For example, within the relatively small genomic region corresponding to the 173 nucleotide fragment amplified by primers set forth in SEQ ID NOs: 16 and 17, it has been found that the human-CAEV virus has at least two mutations that are uniquely human in origin. These mutations correspond to a guanine at position 56 and a cytosine at position 77 within the 173 gag-region amplification product in the Hmc4 clone.

Figure 4 shows a sequence comparision of a PCR amplified 146 nucleotide POL gene region corresponding to nucleotides 3223-3358 of the goat virus CAEV, strain 79-63. The reference strain CAEV-CO comes from the brain of a goat. The brain is an immunologically privileged site. Therefore, the evolution of the virus was different from virus isolated from joint fluid of goats with arthritis. gWa19 was infected with virus from joint fluid, a strain known as CAEV 79-63. The evolution of the human forms should be most similar to CAEV-63. Figure 4 compares a reference goat CAEV-CO, the human-CAEV isolated from Hmc4 and a goat gWa19 CAEV-63 virus. The differences between the goat infecting viral sequences versus the human-infecting CAEV virus in Figure 4 are readily apparent. Thus, novel human-CAEV lentivirae capable of infecting humans are contemplated herein.

Figure 5 shows a direct sequence comparison of the goat-derived CAEV strains CAEV-CO and gWa19 (i.e., CAEV-79-63) and the human-derived h-CAEV strains Hmc1 and Hmc4 for the same 173 nucleotide region set forth in Figure 3. The percentage divergence between CAEV virus isolated from human (human-CAEV) and goats is summarized in Table 4. Hmc1 and Hmc4 correspond to human-CAEV, whereas gWa19, CO and g147 correspond to goat-CAEV.

TABLE 4

| % DIVERGENCE | | | |
|---|---|---|---|
| gag (173 nucleotides) | | | |
| | % max | % min | # of clones |
| Hmc4/gWa19 | 4.0 | 1.7 | 7/1 |
| Hmcl/gWa19 | 8.7 | 1.2 | 10/1 |
| Th8(g147)/gWa19 | 1.7 | 1.7 | 1/1 |
| pol (146 nucleotides) | | | |
| Hmc4/gWa19 | 8.2 | 2.1 | 5/4 |
| gWa19/Co | 4.0 | 4.0 | 1/1 |

(Uniquely human gag mutations include nucleotides at positions 56 and 77).

**EXAMPLE III**

USE OF A CAEV VACCINE TO VACCINATE AN INDIVIDUAL

This example describes a method of administering a CAEV vaccine to an individual in order to stimulate an immune response against CAEV.

Human-CAEV virus is prepared by inoculation of PBMC cells (see Example II) or monocyte cells. Cell supernatants are harvested and CAEV virions are pelleted by centrifugation at 150,000 x g at 4°C to obtain a sterile suspension of approximately 2 x $10^8$ pfu/ml, containing 50% glycerol as a diluent (see Graham et al., J. Infect. Dis. 166: 244-252 (1992), which is incorporated herein by reference). Recipients of live virus are vaccinated intradermally, using a sterile bifurcated needle, with 50 μl CAEV suspension to a single skin site. Vaccinations also can be performed using a killed or attenuated CAEV preparation or other CAEV immunogen such as CAEV gp135 (see, also, Table 1, *supra*). A sterile transparent dressing is applied to the vaccination site, then removed after a crust is formed.

Blood samples are drawn on or about days 14, 28 and 54 after administration of the CAEV immunogen and evaluated to determine antibody titers (humoral response) or T helper or cytotoxic T cell immune response (cellular response). Methods for examining the humoral and cellular responses are disclosed in Example I or otherwise well known in the art (see, for example, Egan et al., J. Infect. Dis. 171:1623-1627 (1995), which is incorporated herein by reference; see, also, Harlow and Lane, *supra,* 1988). If desired, a secondary (booster) immunization is administered on or about day 56 after the initial vaccination. Additional evaluations of humoral and cellular responses are made on or about days 70, 90, 160, 180, 270 and 355 after the initial vaccination. If desired, a tertiary (booster) immunization is administered on or about day 365.

For administration of a CAEV immunogen that is a haptenic peptide, approximately 1 mg of peptide is conjugated to keyhole limpet hemocyanin and administered with an adjuvant, intradermally, as above, in a volume of about 0.1 ml. Evaluations of the humoral and cellular response are made as above and, if desired, secondary or tertiary immunizations are administered.

## Claims

1. A vaccine, comprising live CAEV and a pharmaceutically acceptable carrier.

2. A vaccine, comprising a CAEV immunogen and a pharmaceutically acceptable carrier, wherein said CAEV immunogen is an amino acid sequence selected from the group consisting of CAEV immunogens shown as SEQ ID NOS: 1 to 13.

3. The vaccine of any of claims 1 to 2, further comprising the 70 kDa protein present in the U1 ribonucleoprotein complex, or fragment thereof.

4. Use of a CAEV immunogen for the preparation of a medicament for use in the prophylaxis or treatment of HIV infection.

5. Use of a lymphocyte contacted *in vitro* with a therapeutically effective amount of a CAEV immunogen for the preparation of a medicament for use in the prophylaxis or treatment of HIV infection.

6. The use of claim 5, wherein the lymphocyte is a T cell.

7. The use of claim 5, wherein the lymphocyte is a B cell.

8. The use of claims 4 to 7, wherein the CAEV immunogen is CAEV.

9. The use of claim 8, wherein the CAEV is live CAEV.

10. The use of claim 8, wherein the CAEV is attenuated CAEV.

11. The use of claim 8, wherein the CAEV is killed CAEV.

12. The use of claims 4 to 7, wherein the CAEV immunogen is CAEV gp135.

13. The use of claims 4 to 7, wherein the CAEV immunogen is an amino acid sequence selected from the group consisting of CAEV immunogens shown as SEQ ID NOS: 1 to 13.

14. The use of any of claims 4 to 13, further comprising the 70 kDa protein present in the U1 ribonucleoprotein complex, or fragment thereof.


## Patentansprüche

1. Impfstoff, der einen lebenden CAEV und einen pharmazeutisch verträglichen Trägerstoff umfasst.

2. Impfstoff, der ein CAEV-Immunogen und einen pharmazeutisch verträglichen Trägerstoff umfasst, wobei das CAEV-Immunogen eine Aminosäure-Sequenz aufweist, die aus der Gruppe bestehend aus den als SEQ ID NO: 1 bis SEQ ID NO:13 dargestellten CAEV-Immunogenen ausgewählt ist.

3. Impfstoff nach einem der Ansprüche 1 bis 2, der ferner das im U1 Ribonukleinprotein-Komplex vorliegende 70 kDa-Protein oder ein Fragment desselben umfasst.

**4.** Verwendung eines CAEV-Immunogens zur Herstellung eines Arzneimittels zur Prophylaxe oder Behandlung von HIV-Infektionen.

**5.** Verwendung eines Lymphocyten, der *in vitro* mit einer therapeutisch wirksamen Menge eines CAEV-Immunogens kontaktiert wurde, zur Herstellung eines Arzneimittels zur Verwendung bei der Prophylaxe oder Behandlung von HIV-Infektionen.

**6.** Verwendung nach Anspruch 5, bei der der Lymphocyt eine T-Zelle ist.

**7.** Verwendung nach Anspruch 5, bei der der Lymphocyt eine B-Zelle ist.

**8.** Verwendung nach den Ansprüchen 4 bis 7, bei der das CAEV-Immunogen CAEV ist.

**9.** Verwendung nach Anspruch 8, bei der das CAEV lebendes CAEV ist.

**10.** Verwendung nach Anspruch 8, bei der das CAEV attenuiertes CAEV ist.

**11.** Verwendung nach Anspruch 8, bei der das CAEV ein abgetötetes CAEV ist.

**12.** Verwendung nach den Ansprüchen 4 bis 7, bei der das CAEV-Immunogen CAEV gp135 ist.

**13.** Verwendung nach den Ansprüchen 4 bis 7, bei der das CAEV-Immunogen eine Aminosäure-Sequenz aufweist, die aus der Gruppe bestehend aus den als SEQ ID NO:1 bis SEQ ID NO:13 dargestellten CAEV-Immunogenen ausgewählt ist.

**14.** Verwendung nach einem der Ansprüche 4 bis 13, bei der ferner das im U1 Ribonukleoprotein-Komplex vorliegende 70 kDa-Protein oder ein Fragment desselben umfasst ist.

**Revendications**

**1.** Vaccin comprenant le CAEV vivant et un excipient acceptable d'un point de vue pharmaceutique.

**2.** Vaccin comprenant un immunogène du CAEV et un excipient acceptable d'un point de vue pharmaceutique, dans lequel ledit immunogène du CAEV est une séquence d'acides aminés choisie dans l'ensemble comprenant les immunogènes du CAEV présentées dans les séquences SEQ ID NO: 1 à 3.

**3.** Vaccin selon l'une quelconque des revendications 1 à 2, comprenant en outre la protéine de 70 kDa présente dans le complexe U1-ribonucleoprotéine, ou un de ses fragments.

**4.** Utilisation d'un immunogène du CAEV pour préparer un médicament destiné à être utilisé dans la prophylaxie ou le traitement d'une infection à VIH.

**5.** Utilisation d'un lymphocyte mis en contact *in vitro* avec une quantité à effet thérapeutique d'un immunogène du CAEV pour préparer un médicament destiné à être utilisé dans la prophylaxie ou le traitement d'une infection à VIH.

**6.** Utilisation selon la revendication 5, pour laquelle le lymphocyte est une cellule T.

**7.** Utilisation selon la revendication 5, pour laquelle le lymphocyte est une cellule B.

**8.** Utilisation selon les revendications 4 à 7, pour laquelle l'immunogène du CAEV est un CAEV.

**9.** Utilisation selon la revendication 8, pour laquelle le CAEV est un CAEV vivant.

**10.** Utilisation selon la revendication 8, pour laquelle le CAEV est un CAEV atténué.

**11.** Utilisation selon la revendication 8, pour laquelle le CAEV est un CAEV tué.

**12.** Utilisation selon les revendications 4 à 7, pour laquelle l'immunogène du CAEV est le gp135 du CAEV.

**13.** Utilisation selon les revendications 4 à 7, pour, laquelle l'immunogène du CAEV est une séquence d'acides aminés choisie dans l'ensemble comprenant les immunogènes du CAEV présentés dans les séquences SEQ ID NOS: 1 à 13.

**14.** Utilisation selon l'une quelconque des revendications 4 à 13, comprenant en outre la protéine de 70 kDa présente dans le complexe UI ribonucléoprotéine, ou un de ses fragments.

FIG. 1

FIG. 2

EP 0 910 405 B1

CAEV M33677  G A G C A G T A A G A C A T A T G G C G C A C A G G C C A G G G A A T C C A A T G C T A G T A A A G C A A A A A A C G A A T G A G C C A T A T G A A G

Hmc4(g-30)
Hmc4(g-39)
Hmc4(g-40)
Hmc4(g-46)
Hmc4(g-50)
Hmc4(g-49)
Hmc4(p-254)
Hmc4(p-256)
Hmc4(i-238
Hmc4(i-239)

CAEV M33677  A T T T T G C A G C A A G A C T G C T A G A A G C A A T A G A T G C A G A G C C A G T T A C A C A G C C T A T A A A A G A T T A T C T A A A G C T A A

Hmc4(g-30)
Hmc4(g-39)
Hmc4(g-40)
Hmc4(g-46)
Hmc4(g-50)
Hmc4(g-49)
Hmc4(p-254)
Hmc4(p-256)
Hmc4(i-238
Hmc4(i-239)

CAEV M33677  C A C T A T C T T A T A C A A A T G C A T C A

FIG. 3

EP 0 910 405 B1

```
              3223      3230           3240           3250           3260           3270           3280           3290
Reference    A A G A A C C T A A G C A T C C C G C A A C A G G T T A T T A A A G C A G C G C A A A A A T T A A C C C A A G A A G T C A T C A T T A G G A C A G G A A
Hmc4 (g10)                                           A             G             A             G             A
Hmc4 (g12)                                           A                           A             C             A                       A   T           A           G
Hmc4 (g19)                                           A                           A                 C         A                       A   T       C               G
Hmc4 (g20)                                           A                           A                 C         A                       A   T   C       A           G
Hmc4 (g28)                                           A                           A                 C         A                       A   T           A           G
gWa19 (g51)                                          A             G                           G             A
gWa19 (g52)                                          A             G                           G             A
gWa19 (g53)                                          A             G                           G             A
gWa19 (g60)                                          A             G                           G     G       A

              3300           3310           3320           3330           3340           3350           3360       3368
Reference    A A A T A C C A T G G A T A T T G T T G C C A G G G A A A G A A G A A G A T T G G A G A C T A G A A T T G C A A T T A G G A A C A T C A C
Hmc4 (g10)                           C   A C   A           A
Hmc4 (g12)     G                     G
Hmc4 (g19)                           G       A C
Hmc4 (g20)                           G       A C
Hmc4 (g28)     G                     G       A C
gWa19 (g51)                                  A   A         A
gWa19 (g52)                                  A   A         A
gWa19 (g53)                                  A   A         A
gWa19 (g60)                                  A   A         A                 G
```

FIG. 4

EP 0 910 405 B1

```
                    10           20           30           40           50           60           70
CAEV Co     G A G C A G T A A G A C A T A T G G C G C A C A G G C C A G G G A A T C C A A T G C T A G T A A A G C A A A A A A C G A A T G A G C C A T A T G A A G
Hmc1(g-213)                                 A       G           A           T   A       G           A           G
Hmc4(g-33)                                       A           C       T       G       G G   A           T
gWa19(g-75)                        T     T                   C       T       G       G   A           T

                    80           90          100          110          120          130          140          150
CAEV Co     A T T T T G C A G C A A G A C T G C T A G A A G C A A T A G A T G C A G A G C C A G T T A C A C A G C C T A T A A A A G A T T A T C T A A A G C T A A
Hmc1(g-213)              C                           A           C           A
Hmc4(g-33)   C                       A                   A                           A
gWa19(g-75)  A                       A                   A                           C           G

                   160         170  173
CAEV Co     C A C T A T C T T A T A C A A A T G C A T C A
```

# FIG. 5

30